# EUROPÄISCHE PATENTSCHRIFT

(11) **EP 1 780 288 B1**
(45) Veröffentlichungstag und Bekanntmachung des Hinweises auf die Patenterteilung: **29.10.2008**
(21) Anmeldenummer: 06022257.7
(22) Anmeldetag: 24.10.2006
(51) Int. Cl.: C12Q 1/54, C12Q 1/32, C12Q 1/26, C12Q 1/00, G01N 33/66, G01N 33/52

(54) **Fluoreszenzspektroskopie in absorbierenden Medien**
Fluorescence spectroscopy in absorbing media
Spectroscopie de fluorescence dans des milieux absorbants

(30) Priorität: 25.10.2005 EP 05023318
(43) Veröffentlichungstag der Anmeldung: 02.05.2007
(73) Patentinhaber: F.HOFFMANN-LA ROCHE AG, 4070 Basel (CH); Roche Diagnostics GmbH, 68305 Mannheim (DE)
(72) Erfinder: Petrich, Wolfgang, 76669 Bad Schönborn (DE); Gaessler-Dietsche, Claudia, 69198 Schriesheim (DE); Horn, Carina, 68647 Biblis (DE); Hebestreit, Kai, 69121 Heidelberg (DE); Asfour, Jean-Michel, 69469 Weinheim (DE); Bardelang, Klemens, 68642 Bürstadt (DE); Kocherscheidt, Gerrit, 69126 Heidelberg (DE)
(74) Vertreter: Weiss, Wolfgang

(56) Entgegenhaltungen:
- EP-A- 1 566 637
- GB-A- 2 060 180
- US-A- 6 068 989
- US-A1- 2005 214 891
- SCHWEIZERISCHES ZENTRUM FÜR QUALITÄTSKONTROLLE: "Spektrometrie"[Online] 6. April 2005 (2005-04-06), Seiten 1-4, XP002357846 Gefunden im Internet: URL:http://www.cscq.ch/d/handbuch/programm e/spektrometrie.pdf> [gefunden am 2005-12-06]
- ASAHI R ET AL: "Visible-light photocatalysis in nitrogen-doped titanium oxides." SCIENCE. 13 JUL 2001, Bd. 293, Nr. 5528, 13. Juli 2001 (2001-07-13), Seiten 269-271, XP002357847 ISSN: 0036-8075
- NISHIMURA SUZUSHI ET AL: "Standing wave enhancement of red absorbance and photocurrent in dye-sensitized titanium dioxide photoelectrodes coupled to photonic crystals." JOURNAL OF THE AMERICAN CHEMICAL SOCIETY. 21 MAY 2003, Bd. 125, Nr. 20, 21. Mai 2003 (2003-05-21), Seiten 6306-6310, XP002358669 ISSN: 0002-7863
- HIRAYAMA H. ET AL: 'SHORT WAVELENGTH AND HIGH-EFFICIENCY OPERATION OF DEEP UV LED USING QUATERNARY INALGAN' REZA KENKYU - REVIEW OF LASER ENGINEERING, REZA GAKKAI, OSAKA, JP Bd. 32, Nr. 6, Juni 2004, Seiten 402 - 409, XP009064335

## Beschreibung

Die Erfindung betrifft Verfahren und Vorrichtungen zum Nachweis eines Analyten in einer Probe durch Fluoreszenzmessung.

Messverfahren und -systeme zur biochemischen Analytik sind wichtige Bestandteile der medizinischen Diagnostik. Dabei kann die Bestimmung von Analyten durch Messung des von einem Fluorophoren ausgestrahlten Emissionslichts erfolgen. Um eine genaue und zuverlässige Bestimmung zu ermöglichen, spielt die optimale Wahl der Wellenlänge des für die Erzeugung von Fluoreszenz erforderlichen Anregungslichts eine wichtige Rolle.

Häufig liegen die Anregungsmaxima von Fluorophoren im ultravioletten Spektralbereich (UV). So liegt das langwelligste Anregungsmaximum von NADH beispielsweise bei 340 nm. Allerdings stehen für diesen Spektralbereich derzeit kaum kostengünstige, batteriebetriebene Lichtquellen zur Verfügung und dies auch nur im nahen UV.

Als einzige kostengünstige, schmalbandige Lichtquelle mit niedrigem Stromverbrauch sind für die Anregung im UV derzeit Leuchtdioden von nennenswerter Leistung (>0,1 mW) nur bis hinab zu 365 nm großtechnisch verfügbar, so dass die Anregung nur weitab vom Maximum des Anregungsbereichs stattfinden kann. Neben dem damit einhergehenden Verlust an Fluoreszenzsignal entsteht hierdurch das Problem, dass die Anregungseffizienz sich sehr empfindlich mit der Wellenlänge der LED ändert, da man auf der Flanke des langwelligsten Absorptionspeaks anregt. So beträgt z.B. für NADH die zu erwartende Signaländerung -5 % pro nm im Vergleich zu einer Anregung bei 340 nm. Um eine technische Signalstabilität von z.B. 1 % zu gewährleisten, müsste umgekehrt die Wellenlänge der LED auf 0,2 nm genau stabil bleiben, was aufgrund von Stromschwankungen, Temperaturabhängigkeit und Alterung der LED nur äußerst aufwändig zu bewerkstelligen ist. So würde die Anforderung einer ausreichenden Wellenlängenstabilität lediglich ein sehr kleines Intervall für den erlaubten Temperaturbereich zulassen oder alternativ den Einbau einer aktiven Temperaturregelung in ein Messsystem erfordern, was jedoch aufgrund von Herstellkosten und Stromverbrauch nicht praktikabel wäre.

US-Patent 4,547,465 beschreibt ein Testelement zur Analyse oder zum Transport von Flüssigkeiten, welches eine poröse Zone enthält, die aus einem Polymer mit darin dispergiertem, teilchenförmigem Material, beispielsweise Pigmenten, besteht. Es findet sich jedoch keinerlei Hinweis auf eine Verbesserung der Genauigkeit von Fluoreszenzmessungen.

EP-A-0 066 648 betrifft ein mehrschichtiges Element zur Analytbestimmung in einem wässrigen Medium, das ein Nachweiselement mit einer Nachweisschicht und einer Reaktionsschicht enthält, wobei die Reaktionsschicht ein fasriges, poröses und quellbares Medium enthält. Weiterhin kann das Element eine Lichtabschirmschicht, die teilchenförmige Pigmente enthält, aufweisen. Es findet sich jedoch keinerlei Hinweis auf eine Verbesserung der Genauigkeit von Fluoreszenzbestimmungen.

US 2002/0137027 betrifft eine Verfahren zur Bestimmung von durch eine Oxidase erzeugtem Wasserstoffperoxid mittels eines Lanthanoiden-Liganden-Komplexes. Die Fluoreszenz wird bei einer Wellenlänge von vorzugsweise 330-415 nm angeregt und die Emission bei 600-630 nm nachgewiesen.

US-Patent 3,992,158 beschreibt ein Testelement zur Verwendung bei der Analyse von Flüssigkeiten. Das Testelement kann eine oder mehrere Reflexionsschichten enthalten, die als Absorber Pigmente, wie etwa Titandioxid und Barimsulfat, enthalten. Dieser Reflexionsschicht ist von der die Nachweisreagenzien enthaltende Schicht des Testelements räumlich getrennt. Es findet sich des Weiteren keinerlei Hinweis auf eine Verbesserung der Genauigkeit von Fluoreszenzmessungen.

Die der Erfindung zugrunde liegende Aufgabe bestand darin, ein Verfahren zum Nachweis eines Analyten durch Fluoreszenzmessung bereitzustellen, mit dem die o.g. Nachteile des Standes der Technik zumindest teilweise beseitigt sind. Insbesondere soll ein Verfahren bereitgestellt werden, bei dem die Abhängigkeit des Messsignals von der Anregungswellenlänge reduziert ist.

Die erfindungsgemäße Lösung besteht darin, ein Verfahren zum Nachweis eines Analyten in einer Probe durch Fluoreszenzmessung eines Fluorophors bereitzustellen, wobei dem einen Fluorophor oder eine Vorstufe des Fluorophors enthaltenden Nachweismedium ein Absorber zugegeben wird, dessen Absorptionsspektrum sich mit dem Fluoreszenzanregungsbereich des Fluorophors überlagert. Das im Nachweismedium resultierende System aus Fluorophor und Absorber weist einen effektiven Fluoreszenzanregungsbereich mit einem geänderten effektiven Anregungsmaximum der Fluoreszenz auf. Im Bereich dieses geänderten effektiven Anregungsmaximums kann Fluoreszenz-Anregungslicht eingestrahlt werden. Das durch Bestimmung der Fluoreszenzemission resultierende Messsignal zeigt nur eine geringe Abhängigkeit von der Wellenlänge des Anregungslichts. Ferner können aufgrund der geänderten Wellenlänge des Anregungslichts auch kostengünstige Lichtquellen, wie etwa UV-LEDs, eingesetzt werden.

In einem ersten Aspekt betrifft die vorliegende Erfindung ein Verfahren zum Nachweis eines Analyten in einer Probe durch Fluoreszenzmessung, umfassend die Schritte:
(a) Bereitstellen eines Nachweismediums enthaltend:
   (i) zumindest einen Teil der Probe, in der der Analyt nachgewiesen werden soll,
   (ii) gegebenenfalls mindestens ein Reagenz für den Nachweis des Analyten,
   (iii) einen Fluorophor, der einen Anregungsbereich mit einem Anregungsmaximum im UV-Bereich bei einer ersten Wellenlänge besitzt, oder eine Fluorophor-Vorstufe, aus der in Anwesenheit der Probe und gegebenenfalls der Reagenzien (ii) der Fluorophor gebildet werden kann;
   (iv) einen Absorber, der über einen Teil des Anregungsbereichs des Fluorophors (iii) Licht absorbiert,
      wobei sich für das System aus Fluorophor (iii) und Absorber (iv) ein effektiver Anregungsbereich mit einem effektiven Anregungsmaximum bei einer zweiten, von der ersten verschiedenen Wellenlänge ergibt,
(b) Einstrahlen von Licht zur Anregung des Fluorophors im Bereich der zweiten Wellenlänge,
(c) Bestimmen der Fluoreszenzemission des Fluorophors bei einer oder mehreren geeigneten Messwellenlängen als Nachweis des Vorhandenseins, der Menge oder der Aktivität des Analyten in der Probe, wobei das effektive Anregungsmaximum bei der zweiten Wellenlänge im Spektrum des Fluorophors alleine nicht vorkommt.

In einem weiteren Aspekt betrifft die Erfindung die Verwendung eines Testelements umfassend:
(i) einen Fluorophor, der einen Anregungsbereich mit mindestens einem Anregungsmaximum im UV-Bereich bei einer ersten Wellenlänge besitzt oder eine Fluorophor-Vorstufe, aus der in Anwesenheit der Probe und gegebenenfalls von Reagenzien (iii) der Fluorophor gebildet werden kann;
(ii) einen Absorber, der über einen Teil des Anregungsbereichs des Fluorophors (i) Licht absorbiert,
(iii) gegebenenfalls mindestens ein Reagenz für den Nachweis eines Analyten,
   wobei Fluorophor bzw. Fluorophor-Vorstufe (i) und Absorber (ii) derart auf dem Testelement angeordnet sind, dass zur Anregung des Fluorophors eingestrahltes Licht zuerst auf den Absorber und dann auf den Fluorophor oder im Wesentlichen gleichzeitig auf Fluorophor und Absorber trifft, wobei sich für das System aus Fluorophor (i) und Absorber (ii) ein effektiver Anregungsbereich mit einem effektiven Anregungsmaximum bei einer zweiten Wellenlänge ergibt, wobei das effektive Anregungsmaximum bei der zweiten Wellenlänge im Spektrum des Fluorophors alleine nicht vorkommt,
in einem Verfahren zum Nachweis eines Analyten in einer Probe, umfassend die Schritte:
(a) Inkontaktbringen des Testelements mit der Probe,
(b) Einstrahlen von Licht zur Anregung des Fluorophors im Bereich der zweiten Wellenlänge, und
(c) Bestimmen der Fluoreszenzemission des Fluorophors bei einer geeigneten Messwellenlänge als Nachweis des Vorhandenseins, der Menge oder der Aktivität des Analyten in der Probe.

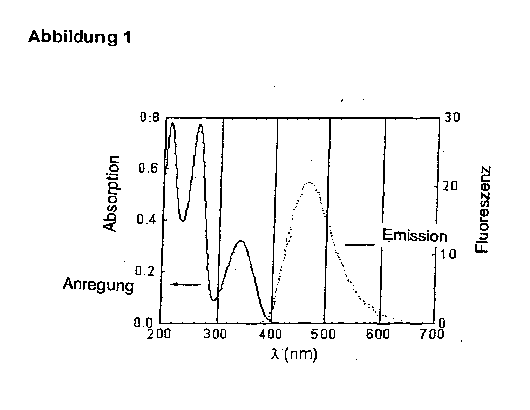

Im Folgenden wird die vorliegende Erfindung durch die Abbildungen 1-6 näher erläutert.

Abbildung 1 zeigt schematisch das Anregungs- und Emissionsspektrum von NADH in wässriger Lösung als Funktion der Wellenlänge λ. Es sind 3 Maxima der Fluoreszenzanregung bei den Wellenlängen 210 nm, 260 nm und 340 nm sowie das Emissionsmaximum im Bereich von 460 nm zu erkennen.

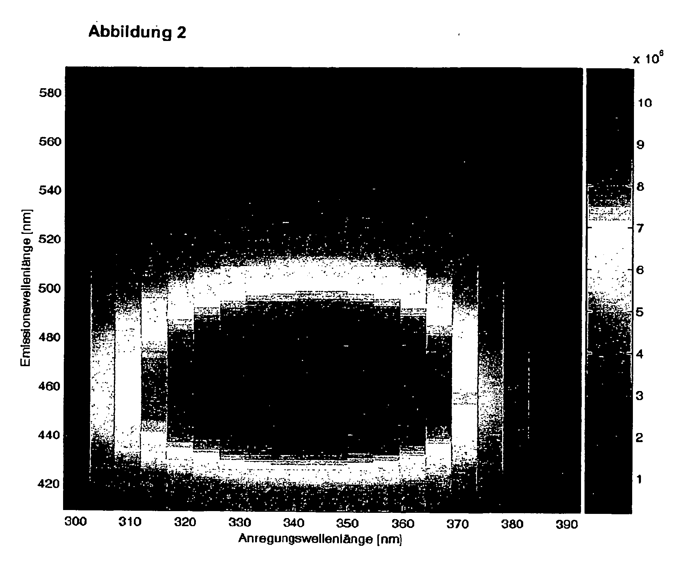

In Abbildung 2 ist die Anregungs-Emissionsmatrix der Fluoreszenzanregung von NADH in 50 mM Hepespuffer, pH 7,5, dargestellt.

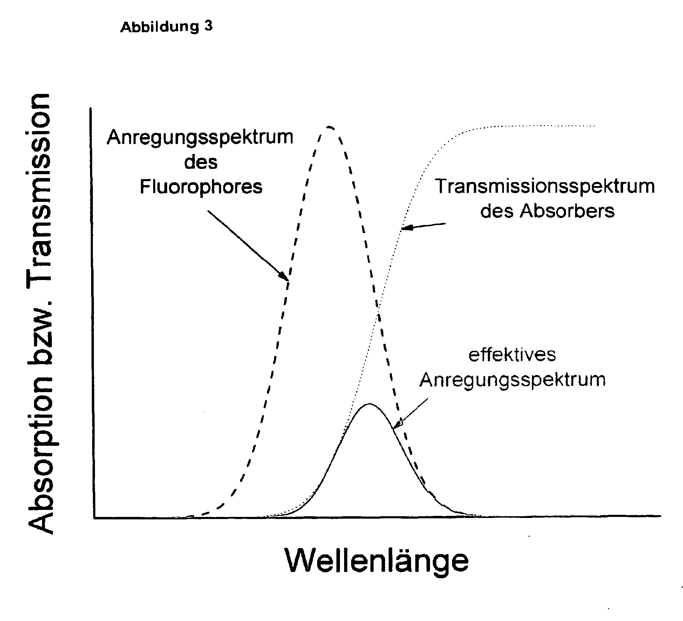

Abbildung 3 zeigt das Schema des Funktionsprinzips der vorliegenden Erfindung. Die Kurve 1 zeigt das Anregungsspektrum eines Fluorophoren in Abwesenheit eines Absorbers. Die Kurve 2 zeigt das Transmissionsspektrum des Absorbers, welches mit dem Anregungsbereich des Fluorophors überlagert. Die Kurve 3 ist der sich aus der Überlagerung des Anregungsbereichs des Fluorophors und des Transmissionsspektrums des Absorbers ergebende effektive Anregungsbereich.

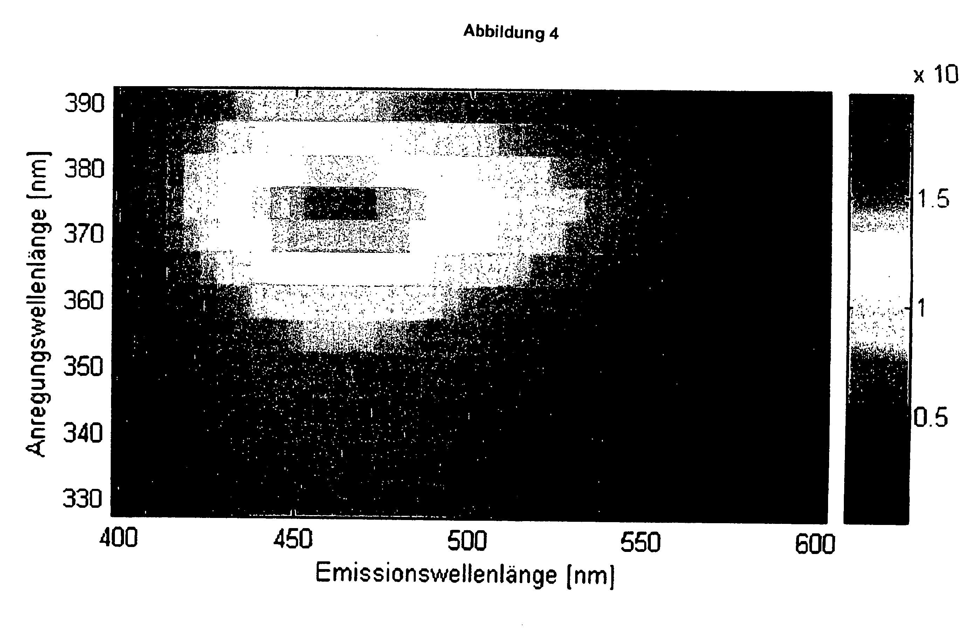

Abbildung 4 zeigt die zu Abbildung 2 analoge Anregungs-Emissionsmatrix für ein Testelement, der NADH als Fluorophor und TiO₂ als Absorber (Rutil, mittlerer Pigmentdurchmesser: 300 nm) enthält. Es ist zu erkennen, dass das Maximum des effektiven Anregungsspektrums im Bereich einer Wellenlänge von 375 nm liegt, für die LEDs kommerziell verfügbar sind. Die Amplitude des effektiven Anregungsspektrums schwankt in dem relevanten Wellenlängenbereich um das Anregungsmaximum um weniger als 1 % pro nm.

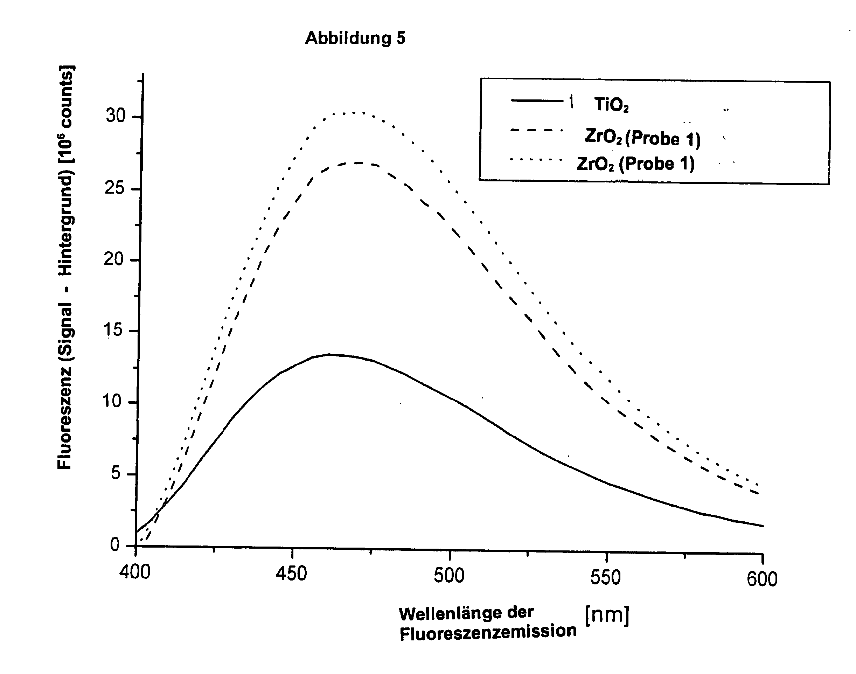

Abbildung 5 zeigt das Emissionsspektrum von NADH unter Verwendung verschiedener Absorber (TiO₂ bzw. ZrO₂ mit 2 unterschiedlichen Partikelgrößen).

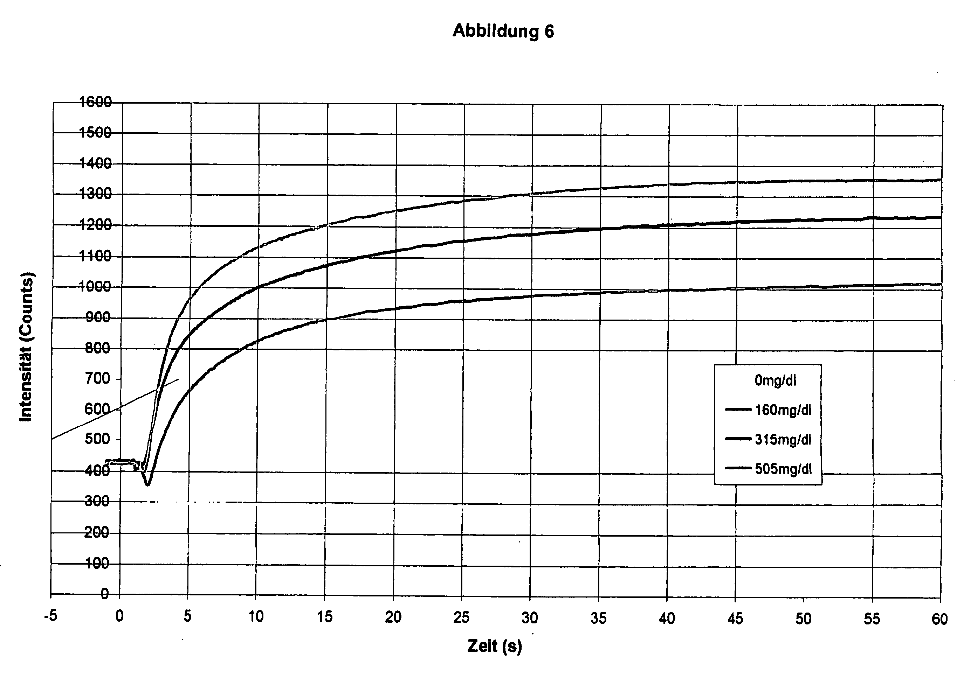

Abbildung 6 stellt die Zeitabhängigkeit der Fluoreszenzsignale von NADH dar. Die Testschicht besteht hierbei aus einem Reagenz und unterschiedlichen Mengen an ZrO₂. Die Anregung findet bei einer Wellenlänge von 375 nm statt und das emittierte Fluoreszenzlicht wird mit einer Photodiode (BPW34) durch einen Kantenfilter (Kunstoffverbundfilter KV418) beobachtet.

Im Sinne der vorliegenden Erfindung bedeutet die Angabe "Anregungsmaximum des Fluorophors" diejenige Wellenlänge, bei der ein System bestehend aus dem Fluorophor in Abwesenheit des Absorbers ein Maximum der Fluoreszenzanregung aufweist. Die Angabe "effektives Anregungsmaximum" bedeutet diejenige Wellenlänge, bei der das gemessene Maximum der Fluoreszenzanregung in einem gegebenen System (Fluorophor und Absorber) liegt. Erfindungsgemäß werden Systeme von Fluorophoren und Absorbern verwendet, die ein geändertes "effektives Anregungsmaximum" aufweisen, d.h. ein Anregungsmaximum, das gegenüber dem Anregungsmaximum des Fluorophors alleine verschoben ist. Eine derartige Verschiebung des Anregungsmaximums ist beispielhaft in Abbildung 3 gezeigt.

Das erfindungsgemäße Verfahren und ein Testelement zur Verwendung in diesem Verfahren können zur Bestimmung beliebiger Analyten, beispielsweise auf dem Gebiet der klinischen Diagnostik, eingesetzt werden. Die Bestimmung des Analyten kann qualitativ oder/und quantitativ erfolgen. Vorzugsweise erfolgt eine quantitative Bestimmung des Analyten, d.h. es wird die Menge, Konzentration oder Aktivität des Analyten in der zu untersuchenden Probe durch Fluoreszenzmessung quantitativ ermittelt.

Als Analyten können mit dem erfindungsgemäßen Verfahren bzw. dem erfindungsgemäß verwendeten Testelement beliebige biologische oder chemische Substanzen bestimmt werden, die durch Fluoreszenzmessung nachgewiesen werden können. Sofern erforderlich, können hierzu neben dem Fluorophor bzw. der Fluorophorvorstufe geeignete Nachweisreagenzien im Verfahren oder dem Testelement eingesetzt werden.

Vorzugsweise ist der Analyt eine durch eine oder mehrere enzymatische Reaktionen bestimmbare Substanz, z.B. ein Enzym oder ein Enzymsubstrat. Bevorzugte Beispiele für den Analyten sind Glucose-Dehydrogenase, Lactat-Dehydrogenase, Malat-Dehydrogenase, Glycerin-Dehydrogenase, Alkohol-Dehydrogenase, α-Hydroxybutyrat-Dehydrogenase, Sorbitol-Dehydrogenase, Aminosäure-Dehydrogenase, Glucose, Milchsäure, Äpfelsäure, Glycerin, Alkohol, Cholesterin, Triglyceride, Lipoproteine, wie LDL oder HDL, Ascorbinsäure, Cystein, Glutathion, Peptide, Harnsäure, Harnstoff, Ammonium, Salicylat, Pyruvat, 5'-Nukleotidase, Creatinkinase (CK), Lactat-Dehydrogenase (LDH) und Kohlendioxid etc.

Beim Nachweis von Enzymsubstraten enthalten die Nachweisreagenzien vorzugsweise ein oder mehrere zum Nachweis des Substrats geeignete Enzyme. Geeignete Enzyme sind z.B. Dehydrogenasen, ausgewählt aus einer Glucose-Dehydrogenase (E.C.1.1.1.47), Lactat-Dehydrogenase (E.C. 1.1.1.27, 1.1.1.28), Malat-Dehydrogenase (E.C.1.1.1.37), Glycerin-Dehydrogenase (E.C.1.1.1.6), Alkohol-Dehydrogenase (E.C.1.1.1.1), α-Hydroxybutyrat-Dehydrogenase, Sorbitol-Dehydrogenase oder Aminosäure-Dehydrogenase, z. B. L-Aminosäure-Dehydrogenase (E.C.1.4.1.5). Weitere geeignete Enzyme sind Oxidasen, wie etwa Glucoseoxidase (E.C.1.1.3.4) oder Cholesterinoxidase (E.C.1.1.3.6) bzw. Aminotransferasen, wie z.B. Aspartat oder Alanin Aminotransferase, 5'-Nukleotidase oder Creatin-Kinase.

Besonders bevorzugt ist der Nachweis von Glucose, wobei das Nachweisreagenz insbesondere Glucose-Dehydrogenase umfasst.

Beim Nachweis von Enzymen enthalten die Nachweisreagenzien vorzugsweise ein oder mehrere zum Nachweis des Enzyms geeignete Substrate.

Weitere Bestandteile der Nachweisreagenzien können übliche Puffer, Hilfs- oder Zusatzstoffe sein.

Im erfindungsgemäßen Verfahren bzw. dem erfindungsgemäß verwendeten System kann als Ausgangsmaterial der Fluorophor selbst eingesetzt werden. Alternativ dazu kann eine Fluorophor-Vorstufe eingesetzt werden, aus der in Anwesenheit der Probe und der Nachweisreagenzien ein Fluorophor gebildet werden kann, dessen Fluoreszenz dann bestimmt wird.

Der Fluorophor ist eine Substanz, die bei Einstrahlung von Fluoreszenz-Anregungslicht ein Messsignal ergibt, welches die Anwesenheit bzw. Abwesenheit des Analyten in der Probe qualitativ anzeigt bzw. das mit der Menge, Konzentration oder Aktivität des Analyten in der Probe korreliert. Beispielsweise kann der Fluorophor selbst der zu bestimmende Analyt sein oder aus den zu bestimmenden Analyten gebildet werden. Vorzugsweise ist jedoch der Fluorophor eine Substanz, bei der es sich um Coenzym einer enzymatischen Reaktion handelt, durch die der Analyt bestimmt wird. Bevorzugte Beispiele für Coenzyme sind Nicotin-Adenin-Dinukleotide, wie etwa NADH oder NADPH, Flavinnukleotide, etc.

Als Fluorophor wird vorzugsweise eine Substanz verwendet, die mindestens ein Anregungsmaximum im UV-Bereich aufweist wie etwa NADH oder NADPH oder Derivate davon.

Als Fluorophor-Vorstufe wird vorzugsweise eine Substanz verwendet, aus der ein Fluorophor gebildet wird, beispielsweise durch eine chemische Reaktion wie etwa eine Oxidation. Bevorzugte Fluorophor-Vorstufen sind Substanzen, aus denen Fluorophore mit mindestens einem Anregungsmaximum im UV-Bereich gebildet werden können, wie etwa NAD oder NADP oder Derivate davon.

Gemäß vorliegender Erfindung wird dem Nachweismedium, welches den Fluorophor bzw. die Fluorophor-Vorstufe und gegebenenfalls mindestens ein weiteres Nachweisreagenz enthält, ein Absorber zugesetzt, dessen Absorptions/Transmissionseigenschaften für in das Nachweismedium eingestrahltes Licht sich über den Anregungsbereich des Fluorophors ändern. Vorzugsweise wird ein Absorber verwendet, der über einen Teil des Anregungsbereichs des Fluorophors Licht absorbiert und über einen anderen Teil des Anregungsbereichs des Fluorophors für Licht weitgehend durchlässig ist.

Besonders bevorzugt wird ein Absorber verwendet, der innerhalb des kürzerwelligen Teils des Anregungsbereichs des Fluorophors Licht absorbiert und innerhalb des längerwelligen Teil des Anregungsbereichs weitgehend im System aus Fluorophor und Absorber lichtdurchlässig ist. Dies führt dazu, dass das effektive Anregungsmaximum im Vergleich zum Anregungsmaximum des Fluorophors in Gegenwart des Absorbers zu einer höheren Wellenlänge verschoben wird. Vorzugsweise liegt das effektive Anregungsmaximum im Vergleich zum Anregungsmaximum des Fluorophors bei einer um mindestens 10 nm, besonders bevorzugt um mindestens 20 nm und am meisten bevorzugt mindestens 30 nm höheren Wellenlänge.

Vorzugsweise wird beim erfindungsgemäßen Verfahren das Licht zur Anregung des Fluorophors im Bereich des geänderten effektiven Anregungsmaximums, z.B. in einem Bereich von ±10 nm, insbesondere von ±5 nm, um die Wellenlänge im Anregungsmaximum des effektiven Anregungsbereichs eingestrahlt. So erfolgt beispielsweise bei Verwendung von NADH oder NADPH als Fluorophor die Anregung der Fluoreszenz bei einer Wellenlänge im Bereich von vorzugsweise 360 nm oder höher, insbesondere von 365-380 nm. Die Anregung der Fluoreszenz erfolgt durch Verwendung einer geeigneten Lichtquelle, beispielsweise durch eine Halogenlampe, eine Leuchtdiode oder eine Laserdiode. Bevorzugt sind Leucht- oder Laserdioden, die Licht in einem Wellenlängenbereich von 370-390 nm abstrahlen. Auf diese Weise ist es möglich, kostengünstige Lichtquellen zur Anregung von Fluoreszenz zu verwenden.

Um die Schaltung eines effektiven Anregungsmaximums für ein System aus Fluorophos und Absorber zu ermöglichen, wird günstigerweise ein Absorber verwendet, mit dem sich die relative Transmission im Nachweismedium für eingestrahltes Licht über den Anregungsbereich des Fluorophors von maximal 20 %, vorzugsweise maximal 10 %, auf mindestens 80 % und vorzugsweise mindestens 90 % bezüglich der maximalen Transmission in dem verwendeten Nachweismedium (Transmission in Abwesenheit des Absorbers) ändert. Die Änderung der relativen Transmission des Nachweismediums erfolgt dabei vorzugsweise innerhalb eines Wellenlängenbereichs von 5100 nm, besonders bevorzugt ≤60 nm und am meisten bevorzugt ≤40 nm.

Als Absorber sind beliebige Substanzen geeignet, welche über einen Teil des Anregungsbereichs des Fluorophors Licht absorbieren und deren Anwesenheit nicht zur Störungen des Nachweisverfahrens führt.

Vorzugsweise liegt der Absorber in Form von Teilchen vor, die einen Durchmesser von ≤1 µm, vorzugsweise ≤500 nm und besonders bevorzugt von 200-400 nm aufweisen. Die Mindestgröße der Teilchen ist vorzugsweise 50 nm. Bevorzugte Beispiele für geeignete Absorbermaterialien sind Metalloxide und Metallsalze, wie etwa Metallsulfide oder Metallsulfate, insbesondere Oxide von Titan, wie etwa TiO, TiO₂, Oxide von Zirkonium, wie ZrO₂, Oxide oder Sulfide von Zink, wie ZnO oder ZnS, und Bariumsalze, wie etwa BaS oder BaSO₄, sowie beliebige Kombinationen davon. Besonders bevorzugt enthält der Absorber TiO₂, welches beispielsweise in der Rutilform vorliegen kann. Grundsätzlich sind Pigmente, die als UV-Blocker in Sonnenschutzcremes oder anderen Formulierungen eingesetzt werden, auch für das erfindungsgemäße Verfahren geeignet.

Bevorzugt kann auch die Verwendung von Absorbermaterialien sein, die lichtstreuende Eigenschaften aufweisen, so dass das Fluoreszenzanregungslicht im Bereich des Nachweismediums mehrfach gestreut und die mittlere Weglänge des Anregungslichts im Nachweismedium vergrößert wird, um eine effizientere Anregung zu erhalten.

Durch Variation des Absorbermaterials, der Korngröße, der Kristallstruktur oder/und der Reinheit, insbesondere durch Zugabe geringerer Mengen weiterer Absorber, kann die Lage und Form des Absorptionsspektrums und damit auch die Form und Lage des Anregungsbereichs des Systems aus Fluorophor und Absorber variiert werden.

Durch geeignete Wahl von Fluorophor oder/und Absorber kann die Flankensteilheit des effektiven Anregungsbereichs des Systems aus Fluorophor und Absorber bei der gewünschten Wellenlänge variiert werden. So kann beispielsweise durch Verwendung von ZrO₂ als Absorber die Absorptionsflanke gegenüber TiO₂ zu kürzeren Wellenlängen hin verschoben werden. Dadurch kann die Amplitude des effektiven Anregungsspektrums aus Fluorophor und Absorber bei der gewünschten Wellenlänge erhöht und trotzdem die Flankensteilheit an dieser Stelle in einem tolerablen Bereich gebracht werden. So zeigt Abbildung 5 das Emissionsspektrum von NADH unter Verwendung von TiO₂ bzw. ZrO₂. Weiterhin kann durch Variation des Absorbers eine Optimierung von Fluoreszenzausbeute und Flankensteilheit der effektiven Absorption erreicht werden.

Die Bestimmung der Fluoreszenzemission des Fluorophors kann auf übliche Weise bei einer oder mehreren geeigneten Messwellenlängen unter Verwendung geeigneter und dem Fachmann bekannter Detektionssysteme erfolgen. So kann die Bestimmung auch durch Messung eines Quenchens der Fluoreszenz, z. B. aufgrund des Vorhandenseins des Analyten erfolgen.

Durch das erfindungsgemäße Verfahren kann die Abhängigkeit des Messsignals von der Wellenlänge des Fluoreszenzanregungslichts deutlich verringert werden. Vorzugsweise wird eine Signalstabilität von ≤ 1% pro nm Änderung der Fluoreszenzanregungswellenlänge erreicht.

Das Verfahren kann als Flüssigtest durchgeführt werden, wobei der Fluorophor bzw. die Fluorophor-Vorstufe, gegebenenfalls mindestens ein weiteres Reagenz und der Absorber in Form einer Suspension in einer wässrigen oder nicht wässrigen Flüssigkeit oder als Pulver vorliegen können. Vorzugsweise wird das Verfahren als Trockentest durchgeführt, wobei das Reagenz auf einem Testelement aufgebracht ist. Das Testelement kann beispielsweise einen Teststreifen oder ein Testband aus saugfähigem oder/und quellbarem Material umfassen, auf das die zu untersuchende Probe aufgebracht wird. Geeignete Materialien können z.B. aus Zellulosen, Kunststoffen etc. ausgewählt werden. Weitere bevorzugte Beispiele für Testelemente sind integrierte Messsysteme, z.B. solche, die ein Element zur Probennahme wie etwa eine Nadel oder Lanzette integriert in Messeinrichtungen und gegebenenfalls Einrichtungen zum Probentransport enthalten. Das Testelement kann ein oder mehrere die Nachweisreagenzien, den Absorber und den Fluorophor oder die Fluorophor-Vorstufe enthaltende Schichten aufweisen. Vorzugsweise sind dabei der Fluorophor bzw. die Fluorophor-Vorstufe und der Absorber auf dem Testelement derart angeordnet, dass zur Anregung des Fluorophors eingestrahltes Licht zuerst auf den Absorber und dann auf den Fluorophor oder gleichzeitig auf Fluorophor und Absorber trifft. Vorzugsweise sind Fluorophor bzw. Fluorophor-Vorstufe und Absorber in einer Schicht auf dem Testelement angeordnet. Bevorzugte Teststreifen sind in EP-A-1035920 beschrieben. Bevorzugte Beispiele für den Aufbau des Testelements als Testband, d.h. als Testelement, welches mehrere Teststreifen enthält, sind in EP-A-1424040 beschrieben, bevorzugte Beispiele für integrierte Messsysteme sind in WO 03/009 759 und WO 2004/107 970 offenbart. Andererseits kann das Nachweisreagenz auch in eine Gelmatrix eingebettet werden (siehe z.B. DE 102 21 845). Auf Offenbarung der zuvor genannten Dokumente wird ausdrücklich Bezug genommen. Besonders bevorzugt ist eine Verfahrensführung, wobei vor dem Aufbringen der Probe der Fluorophor und der Absorber gemeinsam in einer Phase bzw. einer Schicht, z.B. auf einem Testelement, vorliegen.

Die zu untersuchende Probe ist üblicherweise eine flüssige Probe, insbesondere eine Körperflüssigkeit, wie Blut, Plasma, Serum, Speichel oder Urin. Besonders bevorzugt ist die Bestimmung von Glucose in Blut.

Ein weiterer Gegenstand der Erfindung betrifft die Verwendung eines Testelements, wobei das Testelement einen Fluorophor, einen Absorber und gegebenenfalls Nachweisreagenzien enthält, wobei diese Komponenten derart auf dem Testelement angeordnet sind, dass zur Anregung des Fluorophors eingestrahltes Licht zuerst auf den Absorber und dann auf den Fluorophor oder im Wesentlichen gleichzeitig auf Fluorophor und Absorber trifft, wobei sich für das System aus Fluorophor und Absorber ein effektiver Anregungsbereich mit einem effektiven Anregungsmaximum bei einer zweiten Wellenlänge ergibt, wobei das effektive Anregungsmaximum bei der zweiten Wellenlänge im Spektrum des Fluorophors alleine nicht vorkommt, in einem Verfahren zum Nachweis eines Analyten in einer Probe, welches die Schritte umfasst:
(a) Inkontaktbringen des Testelements mit der Probe,
(b) Einstrahlen von Licht zur Anregung des Fluorophors im Bereich einer Wellenlänge, die im Bereich des geänderten effektiven Anregungsmaximums von Fluorophor plus Absorber ist, und
(c) Bestimmen der Fluoreszenzemission des Fluorophors bei einer geeigneten Messwellenlänge als Nachweis des Vorhandenseins bzw. der Menge oder der Aktivität des Analyten In der Probe.

Bevorzugt sind die Komponenten derart angeordnet, dass sie vor dem Aufbringen der Probe in einer Phase bzw. einer Schicht auf dem Testelement vorliegen.

Das Testelement ist vorzugsweise als Teststreifen, Testband oder integriertes Messsystem ausgebildet.

Noch ein weiterer Gegenstand ist die Verwendung eines Absorbers wie zuvor erläutert in einem Testelement zur Schaffung eines effektiven Anregungsmaximums für ein System aus Fluorophor und Absorber, insbesondere in einem Verfahren zur Bestimmung von Analyten wobei der Fluorophor ein Anregungsmaxim im UV-Bereich besitzt und das effektive Anregungsmaximum im Spektrum des Fluorophors alleine nicht vorkommt.

## Patentansprüche

1. Verfahren zum Nachweis eines Analyten in einer Probe durch Fluoreszenzmessung, umfassend die Schritte:
(a) Bereitstellen eines Nachweismediums enthaltend:
(i) zumindest einen Teil der Probe, in der der Analyt nachgewiesen werden soll,
(ii) gegebenenfalls mindestens ein Reagenz für den Nachweis des Analyten,
(iii) einen Fluorophor, der einen Anregungsbereich mit einem Anregungsmaximum im UV-Bereich bei einer ersten Wellenlänge besitzt, oder eine Fluorophor-Vorstufe, aus der in Anwesenheit der Probe und gegebenenfalls der Reagenzien (ii) der Fluorophor gebildet werden kann;
(iv) einen Absorber, der über einen Teil des Anregungsbereichs des Fluorophors (iii) Licht absorbiert,
wobei sich für das System aus Fluorophor (iii) und Absorber (iv) ein effektiver Anregungsbereich mit einem effektiven Anregungsmaximum bei einer zweiten, von der ersten verschiedenen Wellenlänge ergibt,
(b) Einstrahlen von Licht zur Anregung des Fluorophors im Bereich der zweiten Wellenlänge,
(c) Bestimmen der Fluoreszenzemission des Fluorophors bei einer oder mehreren geeigneten Messwellenlängen als Nachweis des Vorhandenseins, der Menge oder Aktivität des Analyten in der Probe, wobei das effektive Anregungsmaximum bei der zweiten Wellenlänge im Spektrum des Fluorophors alleine nicht vorkommt.

2. Verfahren nach Anspruch 1, wobei der Analyt der Fluorophor ist.

3. Verfahren nach Anspruch 1, wobei der Analyt durch eine oder mehrere enzymatische Reaktionen bestimmt wird und der Fluorophor oder die Fluorophor-Vorstufe ein Coenzym einer dieser enzymatischen Reaktionen ist.

4. Verfahren nach Anspruch 3, wobei der Analyt ein Enzym oder ein Enzymsubstrat ist.

5. Verfahren nach einem der vorhergehenden Ansprüche, wobei der Analyt ausgewählt wird aus Glucose-Dehydrogenase, Lactat-Dehydrogenase, Malat-Dehydrogenase, Glycerin-Dehydrogenase, Alkohol-Dehydrogenase, α-Hydroxybutyrat-Dehydrogenase, Sorbitol-Dehydrogenase, Aminosäure-Dehydrogenase, Glucose, Milchsäure, Äpfelsäure, Glycerin, Alkohol, Cholesterin, Triglyceriden, Lipoproteinen, wie LDL oder HDL, Ascorbinsäure, Cystein, Glutathion, Peptiden, Harnsäure, Harnstoff, Ammonium, Salicylat, Pyruvat, 5'-Nukleotidase, Creatinkinase (CK), Lactat-Dehydrogenase (LDH) und Kohlendioxid.

6. Verfahren nach einem der vorhergehenden Ansprüche, wobei Glucose bestimmt wird.

7. Verfahren nach Anspruch 6, wobei die Nachweisreagenzien Glucose-Dehydrogenase umfassen.

8. Verfahren nach einem der vorhergehenden Ansprüche, wobei als Fluorophor NADH oder NADPH oder eine entsprechende Fluorophor-Vorstufe wie etwa NAD oder NADP verwendet wird.

9. Verfahren nach einem der vorhergehenden Ansprüche, wobei das effektive Anregungsmaximum im Vergleich zum Anregungsmaximum des Fluorophors bei einer höheren Wellenlänge, beispielsweise bei um einer mindestens 10 nm, insbesondere um mindestens 20 nm höheren Wellenlänge, liegt.

10. Verfahren nach einem der vorhergehenden Ansprüche, wobei die Anregung der Fluoreszenz bei einer Wellenlänge im Bereich von 360 nm oder höher, insbesondere von 365-380 nm erfolgt.

11. Verfahren nach einem der vorhergehenden Ansprüche, wobei das Einstrahlen von Licht durch eine Leuchtdiode oder eine Laserdiode erfolgt.

12. Verfahren nach einem der vorhergehenden Ansprüche, wobei sich die relative Transmission des Nachweismediums für eingestrahltes Licht über den Anregungsbereich des Fluorophors von maximal 20 % auf mindestens 80 % bezogen auf die maximalen Transmission ändert.

13. Verfahren nach Anspruch 12, wobei die Änderung der relativen Transmission innerhalb eines Wellenlängenbereichs von ≤100 nm, vorzugsweise ≤60 nm und besonders bevorzugt ≤40 nm erfolgt.

14. Verfahren nach einem der vorhergehenden Ansprüche, wobei der Absorber teilchenförmig ist und vorzugsweise einen mittleren Teilchendurchmesser von ≤1 µm, vorzugsweise ≤500 nm und besonders bevorzugt von 200-400 nm aufweist.

15. Verfahren nach einem der vorhergehenden Ansprüche, wobei der Absorber ausgewählt wird aus Metalloxiden und Metallsalzen, wie etwa Sulfiden oder Sulfaten, insbesondere aus TiO, TiO₂, ZrO₂, ZnS, BaS, BaSO₄, ZnO und beliebigen Kombinationen davon.

16. Verfahren nach einem der vorhergehenden Ansprüche, wobei der Absorber weiterhin lichtstreuende Eigenschaften aufweist.

17. Verfahren nach einem der vorhergehenden Ansprüche, wobei eine aus einer Körperflüssigkeit stammende Probe verwendet wird, wie etwa eine Blut-, Plasma-, Serum-, Speichel- oder Urinprobe.

18. Verfahren nach einem der Ansprüche 1 oder 3 bis 17, wobei Fluorophor und Absorber vor Aufbringen der Probe gemeinsam in einer Phase vorliegen.

19. Verfahren nach einem der vorhergehenden Ansprüche, das als Trockentest auf einem Testelement z.B. auf einem Teststreifen oder Testband oder einem integrierten Messsystem mit einem Element zur Probennahme integriert in einer Messeinrichtung durchgeführt wird.

20. Verwendung eines Testelements umfassend
(i) einen Fluorophor, der einen Anregungsbereich mit einem Anregungsmaximum im UV-Bereich bei einer ersten Wellenlänge besitzt, oder eine Fluorophor-Vorstufe, aus der in Anwesenheit der Probe und gegebenenfalls eines Reagenz (iii) der Fluorophor gebildet werden kann;
(ii) einen Absorber, der über einen Teil des Anregungsbereichs des Fluorophors (i) Licht absorbiert,
(iii) gegebenenfalls mindestens ein Reagenz für den Nachweis eines Analyten,
wobei Fluorophor bzw. Fluorophor-Vorstufe (i) und Absorber (ii) derart auf dem Testelement angeordnet sind, dass zur Anregung des Fluorophors eingestrahltes Licht zuerst auf den Absorber und dann auf den Fluorophor oder im Wesentlichen gleichzeitig auf Fluorophor und Absorber trifft, wobei sich für das System aus Fluorophor (i) und Absorber (ii) ein effektiver Anregungsbereich mit einem effektiven Anregungsmaximum bei einer zweiten Wellenlänge ergibt, wobei das effektive Anregungsmaximum bei der zweiten Wellenlänge im Spektrum des Fluorophors alleine nicht vorkommt,
in einem Verfahren zum Nachweis eines Analyten in einer Probe, umfassend die Schritte:
(a) Inkontaktbringen des Testelements mit der Probe,
(b) Einstrahlen von Licht zur Anregung des Fluorophors im Bereich der zweiten Wellenlänge, und
(c) Bestimmen der Fluoreszenzemission des Fluorophors bei einer geeigneten Messwellenlänge als Nachweis des Vorhandenseins, der Menge oder der Aktivität des Analyten in der Probe.

21. Verwendung nach Anspruch 20, wobei das Testelement als Teststreifen, Testband oder integriertes Messsystem mit einem Element zur Probennahme integriert in einer Messeinrichtung ausgebildet ist.

22. Verwendung eines Absorbers in einem Testelement zur Schaffung eines effektiven Anregungsmaximums für ein System aus Fluorophor und Absorber, insbesondere in einem Verfahren zur Bestimmung eines Analyten, wobei der Fluorophor ein Anregungsmaximum im UV-Bereich besitzt und das effektive Anregungsmaximum im Spektrum des Fluorophors alleine nicht vorkommt.

## Claims

1. Method for detecting an analyte in a sample by fluorescence measurement comprising the steps:
(a) providing a detection medium containing:
(i) at least one part of the sample in which the analyte is to be detected,
(ii) optionally at least one reagent for detecting the analyte,
(iii) a fluorophore which has an excitation range with an excitation maximum in the UV range at a first wavelength, or a fluorophore precursor from which the fluorophore can be formed in the presence of the sample and optionally of the reagents (ii);
(iv) an absorber which absorbs light over part of the excitation range of the fluorophore (iii),
resulting in an effective excitation range for the system consisting of fluorophore (iii) and absorber (iv) which has an effective excitation maximum at a second wavelength that is different from the first wavelength,
(b) irradiating with light to excite the fluorophore in the region of the second wavelength,
(c) determining the fluorescence emission of the fluorophore at one or more suitable measuring wavelengths in order to detect the presence, the amount or activity of the analyte in the sample where the effective excitation maximum at the second wavelength does not occur in the spectrum of the fluorophore alone.

2. Method according to claim 1, wherein the analyte is the fluorophore.

3. Method according to claim 1, wherein the analyte is determined by one or more enzymatic reactions and the fluorophore or the fluorophore precursor is a coenzyme of one of these enzymatic reactions.

4. Method according to claim 3, wherein the analyte is an enzyme or an enzyme substrate.

5. Method according to one of the previous claims, wherein the analyte is selected from the group comprising glucose dehydrogenase, lactate dehydrogenase, malate dehydrogenase, glycerol dehydrogenase, alcohol dehydrogenase, α-hydroxybutyrate dehydrogenase, sorbitol dehydrogenase, amino acid dehydrogenase, glucose, lactic acid, malic acid, glycerol, alcohol, cholesterol, triglycerides, lipoproteins such as LDL or HDL, ascorbic acid, cysteine, glutathione, peptides, uric acid, urea, ammonium, salicylate, pyruvate, 5'-nucleotidase, creatine kinase (CK), lactate dehydrogenase (LDH) and carbon dioxide.

6. Method according to one of the previous claims, wherein glucose is determined.

7. Method according to claim 6, wherein the detection reagents comprise glucose dehydrogenase.

8. Method according to one of the previous claims, wherein NADH or NADPH or a corresponding fluorophore precursor such as NAD or NADP is used as the fluorophore.

9. Method according to one of the previous claims, wherein the effective excitation maximum is at a higher wavelength compared to the excitation maximum of the fluorophore, for example a wavelength that is at least 10 nm higher and in particular at least 20 nm higher.

10. Method according to one of the previous claims, wherein the fluorescence excitation occurs at a wavelength in the region of 360 nm or higher, in particular of 365-380 nm.

11. Method according to one of the previous claims, wherein the light is irradiated by a light-emitting diode or a laser diode.

12. Method according to one of the previous claims, wherein the relative transmission of the detection medium for incident light changes across the excitation range of the fluorophore from a maximum of 20 % to at least 80 % based on the maximum transmission.

13. Method according to claim 12, wherein the relative transmission changes within a wavelength range of ≤ 100 nm, preferably ≤ 60 nm and particularly preferably ≤ 40 nm.

14. Method according to one of the previous claims, wherein the absorber is particulate and preferably has an average particle diameter of ≤ 1 µm, preferably of ≤ 500 nm and particularly preferably of 200 - 400 nm.

15. Method according to one of the previous claims, wherein the absorber is selected from metal oxides and metal salts such as sulfides or sulfates and in particular TiO, TiO_{2,} ZrO₂, ZnS, BaS, BaSO₄, ZnO and any combinations thereof.

16. Method according to one of the previous claims, wherein the absorber additionally has light-scattering properties.

17. Method according to one of the previous claims, wherein a sample derived from a body fluid such as a blood, plasma, serum, saliva or urine sample is used.

18. Method according to one of the claims 1 or 3 to 17, wherein the fluorophore and absorber are both present in one phase before applying the sample.

19. Method according to one of the previous claims, which is carried out as a dry test on a test element e.g. on a test strip or test tape or an integrated measuring system comprising one element for sampling integrated into a measuring device.

20. Use of a test element comprising
(i) a fluorophore which has an excitation range with one excitation maximum in the UV range at a first wavelength or a fluorophore precursor from which the fluorophore can be formed in the presence of the sample and optionally of a reagent (iii);
(ii) an absorber which absorbs light over a part of the excitation range of the fluorophore (i),
(iii) optionally at least one reagent for detecting an analyte
wherein the fluorophore or fluorophore precursor (i) and absorber (ii) are arranged on the test element in such a way that light irradiated for the excitation of the fluorophore firstly impinges on the absorber and then on the fluorophore or essentially simultaneously impinges on the fluorophore and absorber resulting in an effective excitation maximum for the system consisting of fluorophore (i) and absorber (ii) at a second wavelength wherein the effective excitation maximum at the second wavelength does not occur in the spectrum of the fluorophore alone,
in a method for detecting an analyte in a sample comprising the steps:
(a) contacting the test element with the sample,
(b) irradiating light to excite the fluorophore in the region of the second wavelength and
(c) determining the fluorescence emission of the fluorophore at a suitable measuring wavelength to detect the presence, the amount or the activity of the analyte in the sample.

21. Use according to claim 20, wherein the test element is designed as a test strip, test tape or integrated measuring system comprising an element for sampling integrated into a measuring device.

22. Use of an absorber in a test element to create an effective excitation maximum for a system consisting of fluorophore and absorber, in particular in a method for determining an analyte wherein the fluorophore has an excitation maximum in the UV range and the effective excitation maximum does not occur in the spectrum of the fluorophore alone.

## Revendications

1. Procédé pour la détection d'un analyte dans un échantillon par mesure de la fluorescence, comprenant les étapes :
(a) mise à disposition d'un milieu de détection contenant :
(i) au moins une partie de l'échantillon, dans lequel l'analyte doit être détecté,
(ii) le cas échéant, au moins un réactif pour la détection de l'analyte,
(iii) un fluorophore, qui possède un domaine d'excitation ayant un maximum d'excitation dans le domaine UV à une première longueur d'onde, ou un précurseur de fluorophore à partir duquel en présence de l'échantillon et, le cas échéant, du réactif (ii), le fluorophore peut être formé ;
(iv) un agent absorbeur, qui absorbe la lumière sur une partie du domaine d'excitation du fluorophore (iii),
étant donné que pour le système fait du fluorophore (iii) et de l'agent absorbeur (iv) il résulte un domaine effectif d'excitation ayant un maximum d'excitation effectif à une deuxième longueur d'onde, différente de la première longueur d'onde,
(b) irradiation de lumière en vue de l'excitation du fluorophore dans le domaine de la seconde longueur d'onde,
(c) détermination de l'émission de fluorescence du fluorophore à une ou plusieurs longueurs d'onde de mesure appropriées en tant que détection de la présence, de la quantité ou de l'activité de l'analyte dans l'échantillon, le maximum d'excitation effectif à la seconde longueur d'onde n'apparaissant pas dans le spectre du fluorophore seul.

2. Procédé selon la revendication 1, dans lequel l'analyte est le fluorophore.

3. Procédé selon la revendication 1, dans lequel l'analyte est déterminé par une ou plusieurs réactions enzymatiques et le fluorophore ou le précurseur de fluorophore est une coenzyme d'une de ces réactions enzymatiques.

4. Procédé selon la revendication 3, dans lequel l'analyte est une enzyme ou un substrat d'enzyme.

5. Procédé selon l'une quelconque des revendications précédentes, dans lequel l'analyte est sélectionné parmi la déhydrogénase du glucose, la déhydrogénase de lactate, la déhydrogénase de malate, la déhydrogénase de la glycérine, la déhydrogénase d'alcools, la déhydrogénase de l'α-hydroxybutyrate, la déhydrogénase du sorbitol, la déhydrogénase d'aminoacides, le glucose, l'acide lactique, l'acide malique, la glycérine, l'alcool, la cholestérine, les triglycérides, les lipoprotéines, comme LDL ou HDL, l'acide ascorbique, la cystéine, le glutathion, les peptides, l'acide urique, l'urée, l'ammonium, le salicylate, le pyruvate, la 5'-nucléotidase, la créatine kinase (CK), la déhydrogénase de lactate (LDH) et le dioxyde de carbone.

6. Procédé selon l'une quelconque des revendications précédentes, dans lequel on détermine le glucose.

7. Procédé selon la revendication 6, dans lequel les réactifs de détection comprenant la déhydrogénase du glucose.

8. Procédé selon l'une quelconque des revendications précédentes, dans lequel le NADH ou le NADPH ou un précurseur de fluorophore correspondant comme, par exemple, le NAD ou le NADP est utilisé en tant que fluorophore.

9. Procédé selon l'une quelconque des revendications précédentes, dans lequel le maximum d'excitation effectif se situe, par comparaison au maximum d'excitation du fluorophore, à une longueur d'onde plus élevée, par exemple, à une longueur d'onde plus élevée d'au moins 10 nm, en particulier, plus élevée d'au moins 20 nm.

10. Procédé selon l'une quelconque des revendications précédentes, dans lequel l'excitation de la fluorescence se fait à une longueur d'onde dans la plage de 360 nm ou au-delà, en particulier de 365-380 nm.

11. Procédé selon l'une quelconque des revendications précédentes, dans lequel l'irradiation de lumière se fait à l'aide d'une diode électroluminescente ou d'une diode laser.

12. Procédé selon l'une quelconque des revendications précédentes, dans lequel la transmission relative du milieu de détection pour la lumière irradiée sur le domaine d'excitation du fluorophore change d'au maximum 20 % à au moins 80 %, par rapport à la transmission maximale.

13. Procédé selon la revendication 12, dans lequel le changement de la transmission relative a lieu au sein d'un domaine de longueurs d'onde de ≤100 nm, de préférence, de ≤60 nm et, en particulier, de préférence, de ≤40 nm.

14. Procédé selon l'une quelconque des revendications précédentes, dans lequel l'agent absorbeur est particulaire et présente, de préférence, un diamètre moyen de particule de ≤1 µm, de préférence, de ≤500 nm et, de manière particulièrement préférée, de 200-400 nm.

15. Procédé selon l'une quelconque des revendications précédentes, dans lequel l'agent absorbeur est sélectionné parmi les oxydes métalliques et les sels métalliques, comme, par exemple, les sulfures ou les sulfates, en particulier parmi TiO, TiO₂, ZrO₂, ZnS, BaS, BaSO₄, ZnO et des combinaisons quelconques de ces derniers.

16. Procédé selon l'une quelconque des revendications précédentes, dans lequel l'agent absorbeur possède en outre des propriétés de dispersion de la lumière.

17. Procédé selon l'une quelconque des revendications précédentes, dans lequel un échantillon provenant d'un liquide corporel est utilisé, comme, par exemple, un échantillon de sang, de plasma, de sérum, de salive ou d'urine.

18. Procédé selon l'une quelconque des revendications précédentes 1 ou 3 à 17, dans lequel le fluorophore et l'agent absorbeur sont présents conjointement dans une phase avant l'apport de l'échantillon.

19. Procédé selon l'une quelconque des revendications précédentes, qui est effectué, intégré dans un dispositif de mesure, en tant que test à l'état sec sur un élément de test, par exemple, sur un ruban de test ou sur une bande de test ou sur un système de mesure intégré avec un élément de prélèvement d'échantillon.

20. Utilisation d'un élément de test comprenant
(i) un fluorophore, qui possède un domaine d'excitation ayant un maximum d'excitation dans le domaine UV à une première longueur d'onde ou un précurseur de fluorophore à partir duquel en présence de l'échantillon et, le cas échéant, d'un réactif (iii), le fluorophore peut être formé ;
(ii) un agent absorbeur, qui absorbe la lumière sur une partie du domaine d'excitation du fluorophore (i) ;
(iii) le cas échéant, au moins un réactif en vue de la détection d'un analyte,
le fluorophore ou le précurseur de fluorophore (i) et l'agent absorbeur (ii) étant disposés sur l'élément de test de telle sorte qu'en vue de l'excitation du fluorophore, de la lumière irradiée tombe tout d'abord sur l'agent absorbeur et alors sur le fluorophore ou pour l'essentiel simultanément sur l'agent absorbeur et sur le fluorophore, étant donné que pour le système fait du fluorophore (i) et de l'agent absorbeur (ii), il résulte un domaine d'excitation effectif ayant un maximum d'excitation effectif à une seconde longueur d'onde, le maximum d'excitation effectif à la seconde longueur d'onde n'apparaissant pas dans le spectre du fluorophore seul,
dans un procédé pour la détection d'un analyte dans un échantillon, comprenant les étapes :
(a) de mise en contact de l'élément de test avec l'échantillon,
(b) d'irradiation de lumière en vue de l'excitation du fluorophore dans la plage de la seconde longueur d'onde, et
(c) de détermination de l'émission de fluorescence du fluorophore dans le cas d'une longueur d'onde de mesure appropriée en tant que détection de la présence, de la quantité ou de l'activité de l'analyte dans l'échantillon.

21. Utilisation selon la revendication 20, dans lequel l'élément de test est réalisé dans un dispositif de mesure en tant que ruban de test, en tant que bande de test ou en tant que système de mesure avec un élément en vue du prélèvement de l'échantillon.

22. Utilisation d'un agent absorbeur dans un élément de test en vue de l'obtention d'un maximum d'excitation effectif pour un système fait d'un fluorophore et d'un agent absorbeur, en particulier dans un procédé pour la détermination d'un analyte, le fluorophore possédant un maximum d'excitation dans le domaine UV et le maximum d'excitation effectif n'apparaissant pas dans le spectre du fluorophore seul.
